# FASCICULE DE BREVET EUROPEEN

(11) **EP 3 280 387 B1**
(45) Date de publication et mention de la délivrance du brevet: **03.07.2019**
(21) Numéro de dépôt: 16715314.7
(22) Date de dépôt: 08.04.2016
(51) Int. Cl.: A61K 8/64, A61Q 19/08, A61Q 19/02

(54) **NOUVELLES UTILISATIONS DU PEPTIDE DE SEQUENCE HIS-D-TRP-ALA-TRP-D-PHE-LYS-NH2 POUR DIMINUER OU RETARDER L'APPARITION DE LA SENESCENCE CELLULAIRE ET DES SIGNES DU VIEILLISSEMENT CUTANE**
NEUARTIGE VERWENDUNGEN DES PEPTIDS DER SEQUENZ HIS-D-TRP-ALA-TRP-D-PHE-LYS-NH2 ZUR VERRINGERUNG ODER VERZÖGERUNG DES AUFTRETENS VON ZELLSENESZENZ UND HAUTALTERUNG
NOVEL USES OF THE PEPTIDE OF SEQUENCE HIS-D-TRP-ALA-TRP-D-PHE-LYS-NH2 FOR REDUCING OR DELAYING THE APPEARANCE OF CELL SENESCENCE AND SIGNS OF SKIN AGEING

(30) Priorité: 10.04.2015 FR 1553139
(43) Date de publication de la demande: 14.02.2018
(73) Titulaire: ISP Investments LLC, Wilmington, DE 19805 (US)
(72) Inventeur: DOMLOGE, Nouha, 06650 Opio (FR); GONDRAN, Catherine, 83440 Callian (FR); MUR, Ludivine, 06580 Pegomas (FR)
(74) Mandataire: Miquel, Corinne
(86) Numéro de dépôt international: PCT/EP2016/057813
(87) Numéro de publication internationale: WO 2016/162512

(56) Documents cités:
- EP-A1- 1 994 939
- EP-A1- 2 666 780
- KR-A-020080 075 530
- DATABASE GNPD [Online] MINTEL; janvier 2012 (2012-01), "Rapid Age Spot and Pigment Lightening Serum", XP002750761, Database accession no. 1719323

## Description

La présente invention se situe dans le domaine des agents actifs cosmétiques et dermato-pharmaceutiques ainsi qu'aux méthodes de traitements cosmétiques s'y rapportant.

La présente invention a pour objet la nouvelle utilisation cosmétique d'une composition comprenant un peptide de synthèse de séquence His-D-Trp-Ala-Trp-D-Phe-Lys-NH2, pour diminuer ou retarder l'apparition de la sénescence cellulaire.

La présente invention a encore pour objet la nouvelle utilisation cosmétique d'une composition comprenant ledit peptide pour diminuer ou retarder l'apparition des signes du vieillissement cutané, et plus particulièrement les rides.

La présente invention a également pour objet une méthode de traitement cosmétique se rapportant à ces nouvelles utilisations, comprenant les étapes d'application de la composition, par voie topique, au moins une fois par jour, pendant une durée d'au moins 2 jours.

La peau est un organe de recouvrement composé de plusieurs couches (derme, jonction dermo-épidermique, épiderme). Le derme est le tissu de soutien de la peau et se compose d'eau, de fibres d'élastine et de fibres de collagène. Ce dernier représente environ 70 % des fibres dermiques et est synthétisé par les fibroblastes.

La synthèse du collagène, sa dégradation et son recyclage sont des étapes essentielles au maintien de son niveau d'expression dans la matrice extracellulaire dermique. Toutes ces étapes sont hautement régulées, notamment le recyclage. Cette dernière étape est réalisée par une enzyme cytoplasmique: la prolidase. La prolidase est un enzyme qui dégrade les imido di et tripeptides contenant de la proline ou de l'hydroxyproline en position C-terminale et provenant principalement du collagène (Surazynski et al. Amino Acids (2008) 35:731-738). Ainsi, grâce à l'action de la prolidase, le stock de proline et d'hydroxyproline dans la cellule est renouvelé et permet la synthèse de nouveau collagène. En effet, le collagène est une protéine composée de 23% de proline et d'hydroxyproline, ainsi la disponibilité des éléments de base que sont la proline et l'hydroxyproline est primordiale à la néosynthèse du collagène (Barbul A., J. Nutr. 138: 2021S-2024S, 2008). 90% de la proline utilisé pour la synthèse du collagène provient de l'action de la prolidase (Son et al., Biol. Pharm. Bull. 30(8) 1395-1399 (2007)).

La peau, comme tous les autres organes, est soumise au processus physiologique complexe du vieillissement. Le vieillissement intrinsèque ou chronologique est la conséquence d'une sénescence génétiquement programmée et d'altérations biochimiques dues à des facteurs endogène. Au niveau de la peau, il se caractérise par un ralentissement de la régénération des cellules et des matrices extracellulaires, aboutissant à une atrophie dermique et épidermique, une sécheresse, une réduction de l'élasticité et de la tonicité de la peau, et l'apparition de ridules et de rides. Le vieillissement extrinsèque, quant à lui, est dû aux agressions environnementales telles que la pollution, le soleil (rayonnements UV), les maladies, les habitudes de vie, etc. Il se superpose au vieillissement intrinsèque au niveau des zones chroniquement exposées à ces agressions; on parle alors de photo-vieillissement.

Ainsi, entre 20 et 80 ans, le nombre de fibroblastes diminuerait de moitié. De plus, lorsque les fibroblastes vieillissent, le taux de synthèse du collagène, ainsi que l'activité de la prolidase diminuent démontrant l'implication de cette enzyme dans la sénescence et l'intérêt d'en faire une cible moléculaire pour lutter contre la sénescence des fibroblastes (Palka et al. Tokai J Exp Clin Med., Vol. 21, No. 4-6, pp. 207- 213, 1996).

Les recherches ont permis d'identifier des agents actifs capables d'augmenter la synthèse du collagène et de lutter contre le vieillissement cutané. Toutefois, il est apparu nécessaire d'identifier de nouveaux composés capables de cibler efficacement la synthèse du collagène au sein de la cellule et de retarder la sénescence cellulaire.

Le peptide de synthèse de séquence His-D-Trp-Ala-Trp-D-Phe-Lys-NH2, dont le nom selon la nomenclature internationale INCI est Hexapeptide-2 est déjà connu pour son activité blanchissante, anti-tâche et éclaircissante de la peau (Sawyer TK et al. Discovery and structure-activity relationships of novel alpha-melanocyte-stimulating hormone inhibitors. Pept Res. 1989 Jan-Feb;2(1) :140-6). De plus la demande KR 10200 8007 5530 A décrit des agents thérapeutiques qui agissent au niveau du récepteur GHS afin d'accélérer la régénération de la peau. Comme agents spécifiques sont mentionnés des peptides ghréline ou encore alternativement le GHRP-2 ou le GHRP-6 (Hexapeptide-2).

Toutefois, son effet sur le vieillissement cutané n'a pas été décrit. Au contraire, la demande de brevet EP 2666780 divulgue que l'Hexapeptide-2 à des concentrations de 1 µM, 10 µM et 100 µM n'a pas d'effets sur la sécrétion de collagène par des fibroblastes cultivés in vitro, alors que le même peptide chimiquement substitué par un groupement biotine augmente la sécrétion de collagène, aux mêmes concentrations.

Néanmoins l'Hexapeptide-2 biotinylé n'a pas montré d'effet sur l'activation de la prolidase et de l'expression du collagène.

En revanche, lors de leurs recherches les inventeurs ont mis en évidence que le peptide de synthèse de séquence His-D-Trp-Ala-Trp-D-Phe-Lys-NH2, lorsqu'il est utilisé à des concentrations plus faibles, comprises entre 0,1 µM et µM :
- augmente l'expression et l'activité de la prolidase,
- augmente l'expression des collagènes de type I et III,
- augmente l'expression du collagène I dans des fibroblastes rendus sénescents,
- diminue ou retarde l'expression de marqueurs de sénescence dans des fibroblastes,
- diminue l'apparence des rides

Ils ont ainsi démontré que ce peptide est adapté pour une utilisation cosmétique visant à diminuer ou retarder l'apparition de la sénescence cellulaire et l'apparition des signes du vieillissement cutané, et plus particulièrement les rides.

Dans le cours de la présente description le peptide de synthèse de séquence His-D-Trp-Ala-Trp-D-Phe-Lys-NH2 sera indifféremment nommé « le peptide de synthèse » ou « le peptide selon l'invention » ou « Hexapeptide-2 ».

Ainsi, l'invention a pour principal objet l'utilisation cosmétique d'une composition comprenant, en tant qu'agent actif, entre 0,1 et 1 µM d'un peptide de synthèse de séquence (SEQ ID N°1) His-D-Trp-Ala-Trp-D-Phe-Lys-NH2 ou un de ses sels dans un milieu physiologiquement adapté, pour diminuer ou retarder l'apparition de la sénescence cellulaire et des signes du vieillissement cutané, tel que revendiqué, la composition étant appliquée par voie topique, au moins une fois par jour, pendant une durée d'au moins 2 jours.

On entend par peau, l'ensemble des tissus de recouvrement constituant la peau, les muqueuses et les phanères.

On entend par l'expression « diminuer ou retarder l'apparition de la sénescence des cellulaire» que le peptide selon l'invention diminue l'expression de marqueurs de sénescence dans des fibroblastes humains dont la sénescence a été induite par extinction du gène FOXO3a ou dont la sénescence réplicative a été induite par un grand nombre de passage en culture ou encore retarde l'apparition de ces mêmes phénomènes.

Par « physiologiquement adapté », on entend des solvants ou des milieux qui conviennent à une mise en contact avec la peau ou les phanères humains, sans risque de toxicité, d'intolérance, d'instabilité, de réponse allergique et autres effets secondaires.

Le peptide selon l'invention dilué est ensuite stérilisé par filtration stérile.

Par « signes du vieillissement cutané », on entend les modifications de l'aspect extérieur de la peau et des phanères dues au vieillissement qui sont l'amincissement de la peau, le relâchement, la perte d'hydratation, l'atonie, les rides profondes et les ridules, la perte de fermeté et de tonicité, l'atrophie dermique ou toute autre dégradation interne de la peau, à l'exception des anomalies pigmentaires de la peau comme les tâches de vieillesse ou lentigo ou toute autre irrégularité du teint.

Préférentiellement, on entend par l'expression « diminuer les signes du vieillissement cutané » que le peptide selon l'invention diminue le nombre, la surface totale, la longueur totale et la profondeur totale des rides.

Avantageusement, les compositions selon l'invention se présentent sous une forme adaptée à l'application par voie topique.

Par application topique, on désigne le fait d'appliquer ou d'étaler la composition comprenant le peptide selon l'invention, à la surface de la peau ou d'une muqueuse.

Avantageusement, la composition comprenant le peptide selon l'invention en tant qu'agent actif, sera appliquée par voie topique à la fréquence d'au moins une fois par jour.

Dans un autre mode de réalisation la composition sera appliquée à la fréquence de 2 fois par jour.

Avantageusement, la composition comprenant le peptide selon l'invention en tant qu'agent actif, sera appliquée par voie topique pendant une durée d'au moins 2 jours.

Dans un autre mode de réalisation la composition sera appliquée pendant une durée d'au moins 6 semaines.

Dans un autre mode de réalisation la composition sera appliquée pendant une durée d'au moins 3 mois.

Le peptide selon l'invention est un hexapeptide de synthèse de séquence His-D-Trp-Ala-Trp-D-Phe-Lys-NH2. Préférentiellement le peptide est sous forme de sel de trifluoroacétate.

Le peptide selon l'invention est avantageusement solubilisé dans un ou plusieurs solvants physiologiquement adaptés, tels que l'eau, le glycérol, l'éthanol, le propanediol, le butylène glycol, le dipropylène glycol, les diglycols éthoxylés ou propoxylés, les polyols cycliques ou tout mélange de ces solvants.

Avantageusement, le peptide selon l'invention est en solution dans de l'eau.

Après cette étape de dilution, le peptide selon l'invention peut être encapsulé ou inclus dans un vecteur cosmétique tels que les liposomes ou toute autre microcapsule utilisée dans le domaine de la cosmétique ou adsorbé sur des polymères organiques poudreux, des supports minéraux comme les talcs et bentonites, et plus généralement solubilisé dans, ou fixé sur, tout vecteur physiologiquement adapté.

La composition utilisable selon l'invention pourra être appliquée par toute voie appropriée, notamment orale, ou topique externe, et la formulation des compositions sera adaptée par l'homme du métier.

Préférentiellement, les compositions selon l'invention se présentent sous une forme adaptée à l'application par voie topique. Ces compositions doivent donc contenir un milieu physiologiquement acceptable, c'est-à-dire compatible avec la peau et les phanères, sans risque d'inconfort lors de leur application et couvrent toutes les formes cosmétiques.

Ces compositions pourront notamment se présenter sous forme d'une solution aqueuse, hydro-alcoolique ou huileuse, d'une émulsion huile-dans-eau, eau-dans-huile ou émulsions multiples, de gel aqueux ou anhydre, de colloïde. Ces compositions peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'une crème, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des co-solvants (éthanol, glycérol, alcool benzylique, humectant...), des épaississants, des diluants, des émulsionnants, des antioxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut, par exemple, citer des polymères hydrosolubles de type polymère naturel, tels que les polysaccharides, ou polypeptides, des dérivés cellulosiques de type méthylcellulose ou hydroxypropylcellulose, ou encore des polymères synthétiques, polaxamères, carbomères, siloxanes, PVA ou PVP, et notamment les polymères vendus par la société Ashland.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être présents à des concentrations allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Il est bien entendu que le peptide selon l'invention, en tant qu'agent actif, peut être utilisé seul ou bien en association avec d'autres agents actifs.

Avantageusement, les compositions utilisables selon l'invention contiennent, en outre, au moins un autre agent actif destiné à renforcer l'action de l'agent actif selon l'invention, dans le domaine de la prévention et de l'amélioration des signes du vieillissement cutané, ou encore un autre agent actif permettant d'élargir la gamme de propriétés de la composition considérée.

On peut citer, de manière non limitative, les classes d'ingrédients suivantes : les agents régénérants, anti-âges, antirides, apaisants, anti-radicalaires, anti-glycation, hydratants, antibactériens, antifongiques, kératolytiques, myorelaxants, desquamants, raffermissants, les agents stimulant la synthèse de macromolécules dermiques ou le métabolisme énergétique ou la microcirculation ou la pousse des ongles ou la pousse des cheveux, les agents modulant la différenciation ou la pigmentation de l'épiderme, les agents inhibant les métallo-protéinases, ou encore les écrans ou filtres solaires.

Dans un mode plus particulier de réalisation, la composition selon l'invention comprendra, outre le peptide selon l'invention,
- au moins un composé activateur du cytochrome c et/ou,
- au moins un composé hydratant, tel qu'un composé activateur des aquaporines et/ou,
- au moins un composé activateur des sirtuines et/ou,
- au moins un composé qui augmente l'adhésion cellulaire et/ou,
- au moins un composé qui augmente la production de protéines matricielles telles que le collagène, fibronectine, laminine, glycosaminoglycanes et/ou,
- au moins un composé modulateur de l'activité du protéasome et/ou,
- au moins un composé modulateur du rythme circadien et/ou,
- au moins un composé modulateur des protéines HSP et/ou,
- au moins un composé qui augmente l'énergie cellulaire et/ou,
- au moins un composé modulant la pigmentation de la peau et/ou,
- au moins un composé activateur du coenzyme Q10 et/ou,
- au moins un composé améliorant la fonction barrière, tel qu'un composé activateur de la transglutaminase ou de la HMG-CoA réductase et/ou,
- au moins un composé protecteur de la mitochondrie.

Lesdits composés ci-dessus peuvent être d'origine naturelle, comme des hydrolysats peptidiques de plantes, ou encore d'origine synthétique, comme des peptides.

Indépendamment de leurs fonctions, les autres agents actifs associés à l'agent actif selon l'invention dans la composition pourront avoir des structures chimiques très diverses. On peut citer de manière non limitative, les peptides, la vitamine C et ses dérivés, les vitamines du groupe B, la DHEA (dihydroépiandrostérone), les phytostérols, l'acide salicylique et ses dérivés, les rétinoïdes, les flavonoïdes, les amines de sucres, les azoles, les sels métalliques, les extraits peptidiques d'origine végétale ou encore les polymères.

Avantageusement, le peptide selon l'invention est présent dans la composition à une concentration comprise entre 0,1 et 1 µM, préférentiellement à une concentration comprise entre 0,5 et 1 µM et encore plus préférentiellement à une concentration inférieure strictement à 1 µM.
Sans n'être aucunement lié par une théorie scientifique, cette gamme de concentrations représente la quantité nécessaire de peptide selon l'invention, en tant qu'agent actif, pour obtenir l'effet moléculaire recherché, défini comme l'augmentation optimale de l'activité de la prolidase et de la synthèse du collagène.

Avantageusement, cette gamme de concentration permet l'augmentation maximum de la synthèse des collagènes de type I et III.

L'invention et les avantages qui en découlent seront mieux compris à la lecture des modes de réalisation non limitatifs qui suivent, rédigée au regard des figures annexées dans lesquelles :
- la Figure 1 représente l'étude de l'expression de miARN-29a 3p dans des fibroblastes humains provenant de donneurs d'âges différents;
- la Figure 2 représente l'évaluation de l'effet de l'Hexapeptide-2 sur l'expression de collagène I dans des fibroblastes humains rendus sénescents par transfection avec le siRNA FOXO3a ;
- la Figure 3 représente l'évaluation de l'effet de l'Hexapeptide-2 sur l'activité de la β-galactosidase dans des fibroblastes humains rendus sénescents par sénescence réplicative (traitement long terme) ;
- la Figure 4 représente l'évaluation de l'effet de l'Hexapeptide-2 sur l'activité de la β-galactosidase dans des fibroblastes humains sénescents par sénescence réplicative, en présence de Hexapeptide-2 (traitement court terme);
- la Figure 5 représente l'évaluation de l'effet de l'Hexapeptide-2 sur l'activité de la prolidase dans des fibroblastes humains rendus sénescents par sénescence réplicative (traitement long terme) ;
- la Figure 6 représente l'évaluation de l'effet de l'Hexapeptide-2 sur l'expression de miARN-29a 3p dans des fibroblastes humains rendus sénescents par sénescence réplicative (traitement long terme);
- la Figure 7 représente l'effet de l'Hexapeptide-2 à 1 % sur la surface totale des rides évaluée par étude clinique

Dans la description et les exemples, à moins qu'il ne soit spécifié autrement, il est entendu que, lorsqu'un intervalle est donné, il inclut les bornes supérieure et inférieure dudit intervalle.

### Exemple 1 : Etude de l'expression de la prolidase dans des fibroblastes humains, en présence d'Hexapeptide-2

Le but de cette étude est de déterminer l'influence de L'Hexapeptide-2 sur l'expression de la prolidase dans des fibroblastes humains.

Protocole : Des fibroblastes humains en culture sont traités avec de l'Hexapeptide-2 à une concentration finale de 0,5 et 1 µM après dilution dans du milieu de culture, pendant 24 heures (2 applications par jour). Les cellules sont ensuite lavées, fixées au méthanol froid pendant 4 minutes à 4°C. Les cellules sont incubées en présence d'un anticorps monoclonal de souris spécifique de la prolidase (LifeSpan BioSciences, Réf. LS-C115644), puis d'un anticorps secondaire anti-souris couplé à un fluorochrome (Invitrogen, Réf. A21202). Les cellules sont alors examinées au microscope à Epi-fluorescence (Zeiss Axiovert 200M microscope). Une quantification de la fluorescence, à partir des photographies obtenues, a été réalisée.

Résultats : Les observations microscopiques représentées dans le tableau 1 montrent une fluorescence cytoplasmique significativement plus intense dans les cellules traitées par de l'Hexapeptide-2 (selon le test t de Student).

| Tableau 1 | Non traité | 0.5 µM Hexapeptide-2 | 1 µM Hexapeptide- 2 |
|---|---|---|---|
| Expression de la prolidase (intensité / aire des cellules (%)) | 100 | 118 | 128 |

Conclusion : L'Hexapeptide-2 stimule l'expression de la prolidase dans les fibroblastes humains.

### Exemple 2 : Etude de l'activité de la prolidase dans des fibroblastes humains, en présence d'Hexapeptide-2

Le but de cette étude est de déterminer l'influence de l'Hexapeptide-2 sur l'activité de la prolidase dans des fibroblastes humains.

Protocole : Des fibroblastes humains en culture sont traités avec de l'Hexapeptide-2 à une concentration de 0,5 et 1 µM après dilution dans du milieu de culture, pendant 24 heures (2 applications par jour). En parallèle, des fibroblastes ont été traités avec un activateur et un inhibiteur de la prolidase : l'acide rétinoïque (Sigma, Réf. R2625) et le Cpz-Pro (Bachem, Réf. 4001343). Puis, les cellules sont décrochée de la boîte à l'aide d'une solution de NaCl à 150 mM. Après avoir récupéré le culot cellulaire et activé la prolidase par une solution contenant du MnCl₂ à 2 mM pendant 2 heures à 37°C, le substrat de la prolidase est ajouté : 47 mM de Glycine-Proline (Sigma, Réf. G3002) pendant 1 heure à 37°C. Puis, le taux de proline contenu dans chaque échantillon est déterminé à une longueur d'onde de 515 nm par le réactif de Chinard. En parallèle, une gamme de concentration de proline est effectuée permettant l'établissement d'une courbe de calibration. Le taux de protéine est déterminé en utilisant le kit dosage de protéine BCA (Thermo Scientific, Réf. 23225), permettant de reporter le taux de proline sur le nombre de protéines de chaque échantillon.

Résultat : Le traitement avec l'Hexapeptide-2 permet d'augmenter significativement l'activité de la prolidase (selon le test t de Student). Les résultats sont présentés dans le tableau 2 ci-dessous.

| Tableau 2 | Non traité | 0.5 µM Hexapeptide-2 | 1 µM Hexapeptide-2 | 1µM Acide rétinoïque | 10mM Cpz-Pro |
|---|---|---|---|---|---|
| Activité de la prolidase (%) | 100 | 122 | 114 | 105 | 90 |

Conclusion : L'Hexapeptide-2 stimule l'activité de la prolidase dans les fibroblastes humains.

### Exemple 3 : Etude de l'expression du collagène I intracellulaire dans des fibroblastes humains, en présence d'Hexapeptide-2

Le but de cette étude est de déterminer l'influence de l'Hexapeptide-2 sur l'expression du collagène I intracellulaire dans des fibroblastes humains par cytométrie de flux.

Protocole : Des fibroblastes humains en culture sont traités avec l'Hexapeptide-2 à une concentration de 0,5 et 1 µM après dilution dans du milieu de culture, pendant 48 heures (2 applications par jour). En parallèle, des fibroblastes ont été traités avec un activateur du collagène I : le TGF-β (R&D system, Réf. 240B). Les fibroblastes sont trypsinisés et le culot cellulaire récupéré. Les cellules sont ensuite fixées au méthanol froid pendant 15 minutes à 4°C. L'immunomarquage est réalisé à l'aide d'un anticorps monoclonal de souris spécifique du collagène I (Millipore, Ref : anti-Collagène Type I, Clone: 5D8-G9) couplé à un fluorochrome. Puis, 10 000 cellules sont analysées au cytomètre de flux (Guava EasyCyte) afin de déterminer le taux de fluorescence dans chacune d'elles.

Résultats : Suite à l'analyse par cytométrie de flux, une fluorescence cytoplasmique plus intense a été détectée dans les cellules traitées par l'Hexapeptide-2. Les résultats sont présentés dans le tableau 3 ci-dessous.

| Tableau 3 | Non traité | 0.5 µM Hexapeptide-2 | 1 µM Hexapeptide-2 | 10 ng/ml TGF-β |
|---|---|---|---|---|
| Expression du collagène I (%) | 100 | 120 | 145 | 182 |

Conclusion : L'Hexapeptide-2 stimule l'expression du collagène I dans les fibroblastes humains.

### Exemple 4 : Etude de l'expression des collagènes I et III dans la peau humaine, en cultivée ex vivo en présence d'Hexapeptide-2

Le but de cette étude est de déterminer l'influence de l'Hexapeptide-2 sur l'expression des collagènes I et III dans la peau *ex vivo* humaine.

Protocole : Des échantillons de peau humaine sont mis en culture à l'interface air/liquide. L'Hexapeptide-2 est appliqué topiquement sur les échantillons à des concentrations de 0,5 and 1 µM après dilution dans du milieu de culture, à raison de 2 applications par jour, puis les échantillons sont incubés durant 48 heures à 37°C.
Ces échantillons de peau sont ensuite fixés avec du formaldéhyde puis inclus dans de la paraffine. Des coupes de 4 µm sont ensuite réalisées.
Les marquages des collagènes I et III sont effectués après démasquage des sites spécifiques par une incubation au micro-onde puis par un traitement à la trypsine. Les immunomarquages sont réalisés à l'aide d'un anticorps polyclonal de lapin spécifique du collagène I (Rockland, Réf. 600-401-103-0.5), d'un anticorps polyclonal de lapin spécifique du collagène III (Rockland, Réf. 600-401-105-0.5), puis d'un anticorps secondaire anti-lapin couplé à un fluorochrome (Invitrogen, Réf. A21206). Les cellules sont alors examinées au microscope à Epi-fluorescence (Zeiss Axiovert 200M microscope). Une quantification de la fluorescence, à partir des photographies obtenues, a été réalisée.

Résultats : Les observations microscopiques montrent une fluorescence significativement plus intense au niveau du derme des biopsies traitées avec l'Hexapeptide-2. Les résultats sont présentés dans le tableau 4 ci-dessous.

| Tableau 4 | Non traité | 0.5 µM Hexapeptide-2 | 1 µM Hexapeptide-2 |
|---|---|---|---|
| Expression du Collagène I (%) | 100 | 119 | 154 |
| Expression du Collagène III (%) | 100 | 155 | 155 |

Conclusions : L'Hexapeptide-2 stimule l'expression des collagènes I et III dans la peau *ex vivo* humaine.

### Exemple 5 : Etude de l'expression de miARN-29a 3p dans des fibroblastes humains provenant de donneurs d'âges différents

Le but de cette étude est de déterminer l'influence de l'Hexapeptide-2 sur l'expression de miARN-29a 3p dans des fibroblastes humains provenant de donneurs d'âges différents, par qPCR. Les miARNs jouent un rôle fondamental dans la régulation post-transcriptionnelle de leurs cibles en les inhibant. Il a été démontré, dans la littérature, que l'expression du miARN-29a augmente avec la sénescence (Mancini M. *et al*., 2014). Grâce à des bases de données telles que miRanda, les cibles du miARN-29a ont été identifiées dont le collagène I et la prolidase.

Protocole : Des fibroblastes humains provenant de 3 donneurs âgés de 33, 45 et 66 ans sont traités avec de l'Hexapeptide-2 à une concentration de 0,5 et 1 µM dans du milieu de culture, pendant toute la nuit (1 application).
Les miARNs sont extraits à l'aide d'un kit d'extraction (Ambion, Réf. AM1561), puis reverse transcrit avec un kit spécifique (Applied Biosystem, Réf. 4374966). Une PCR en temps réel est réalisée dans un thermocycleur à l'aide d'un *TaqMan Gene Expression Assays* spécifique du miARN-29a 3p (Applied Biosystem, Réf. 002112) et d'un *TaqMan Gene Expression Assays* spécifique du RNU44 utilisé comme contrôle endogène (Applied Biosystem, Réf. 001094). La quantification relative de l'expression du miARN-29a 3p est réalisée par la méthode comparative des Ct.

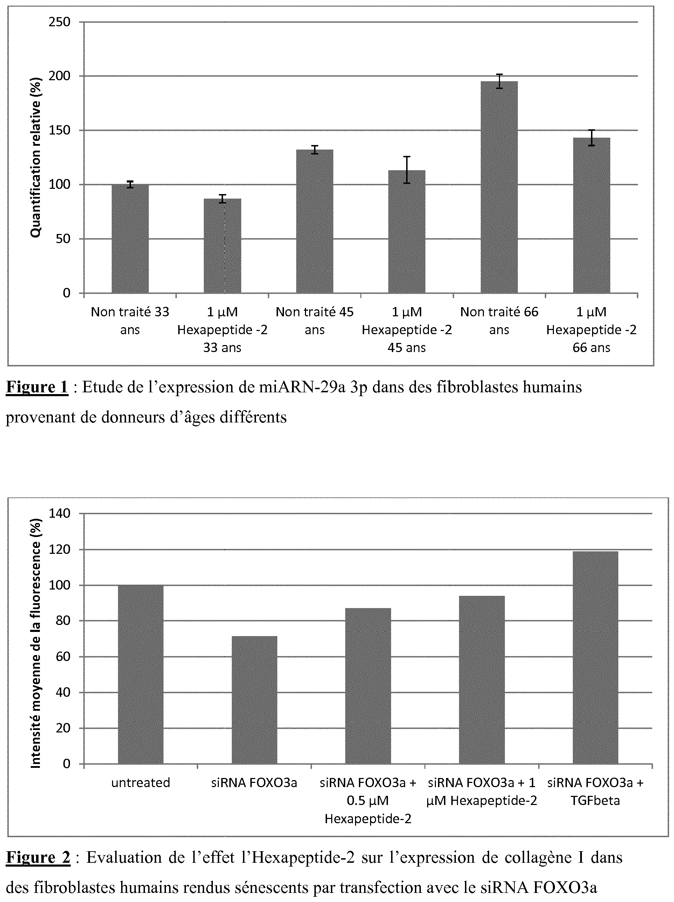

Résultat : Suite à la comparaison de trois âges, l'expression de miARN-29a 3p augmente significativement de +32% (entre 33 et 45 ans) et de +48% (entre 45 et 66 ans) (test *t* de Student) avec la sénescence. A chaque âge ; 33, 45 et 66 ans, les cellules traitées avec l'Hexapeptide-2 montrent, respectivement, une diminution significative de -13%, -14% et de -27% de l'expression de miARN-29a 3p (selon le test *t* de Student). Les résultats sont présentés dans la figure 1.

Conclusion : L'expression de miARN-29a 3p est diminuée lorsque les cellules sont traitées avec Hexapeptide-2, ce qui permet de limiter l'impact négatif de son expression sur les cibles telles que le collagène et la prolidase.

### Exemple 6 : Etude de l'activité de la β-galactosidase dans des fibroblastes humains rendus sénescents par transfection avec le siRNA FOXO3a, en présence d'Hexapeptide-2

Le but de cette étude est de déterminer l'influence de l'Hexapeptide-2 sur des fibroblastes humains sénescents. La sénescence a été induite par l'inhibition du gène FOXO3a grâce à l'ARN interférant (siRNA) de FOXO3a. FOXO3a est un facteur de transcription de type Forkhead impliqué dans la longévité cellulaire. En inhibant l'expression de FOXO3a, les cellules sur-expriment la β-galactosidase, caractéristique des cellules sénescentes.

Protocole : Des fibroblastes humains en culture sont traités ou non avec un siRNA spécifique de FOXO3a (Invitrogen, Réf. HSS177176) à une concentration finale de 25 nM en utilisant la technique de transfection par la *Lipofectamine*™ *RNAiMAX* (Invitrogen, Réf. 56531) et traités ou non avec de l'Hexapeptide-2 à une concentration finale de 1 µM dans du milieu de culture, pendant 48 heures (2 applications par jour).
Les cellules sont rincées et fixées dans un tampon de fixation (0.2% glutaraldéhyde, 2% formaldéhyde). Les cellules sont ensuite incubées, à 37°C sans CO₂ pendant 24 heures, avec une solution de X-Gal à 1 mg/ml dans 40 mM d'acide citrique/phosphate (pH 6), 5 mM K₃FeCN₆, 5 mM K₄FeCN₆, 150 mM NaCl and 2 mM MgCl₂. Les cellules sont alors examinées au microscope à lumière blanche (Nikon Eclipse E600 microscope).

Résultats : Les cellules sénescentes ayant une activité β-galactosidase spécifique sont colorées en bleu. Les cellules traitées avec le siRNA spécifique de FOXO3a montrent une augmentation de l'activité β-galactosidase liée à la sénescence, se traduisant par une augmentation du nombre de cellules colorées en bleue. Les cellules transfectées avec les siRNA spécifique de FOXO3a et traitées avec l'Hexapeptide-2 montrent une diminution visible de l'activité de la β-galactosidase liée à la sénescence, se traduisant par une diminution du nombre de cellules colorées en bleue (résultats non montrés).

Conclusion : Le traitement avec Hexapeptide-2 permet d'observer des cellules dont le niveau de sénescence est moindre que les cellules transfectées non traitées.

### Exemple 7 : Etude de l'expression de collagène I dans des fibroblastes humains rendus sénescents par transfection avec le siRNA FOXO3a, en présence d'Hexapeptide-2

Le but de cette étude est de déterminer l'influence de l'Hexapeptide-2 sur l'expression du collagène I dans des fibroblastes humains sénescents par transfection avec le siRNA FOXO3a.

Protocole : Des fibroblastes humains en culture sont traités ou non avec un siRNA spécifique de FOXO3a (Invitrogen, Réf. HSS177176) à une concentration finale de 25 nM en utilisant la technique de transfection par la *Lipofectamine*™ *RNAiMAX* (Invitrogen, Réf. 56531) et traités ou non avec de l'Hexapeptide-2 à une concentration de 0,5 ou 1 µM dans du milieu de culture, pendant 48 heures (2 applications par jour).
Les fibroblastes sont trypsinisés et le culot cellulaire récupéré. Les cellules sont ensuite fixées au méthanol froid pendant 15 minutes à 4°C. L'immunomarquage est réalisé à l'aide d'un anticorps monoclonal de souris spécifique du collagène I (Millipore, Réf. anti-Collagène Type I, Clone: 5D8-G9) couplé à un fluorochrome. Puis, 10 000 cellules sont analysées au cytomètre de flux (Guava EasyCyte) afin de déterminer le taux de fluorescence dans chacune d'elles.

Résultats : Suite à l'induction de la sénescence par la transfection avec le siRNA FOXO3a, une diminution l'expression de collagène I est observée dans ces cellules. Les cellules sénescentes et traitées avec l'Hexapeptide-2 présentent quant à elles, une diminution moindre de l'expression de collagène I. Les résultats sont présentés dans la figure 2.

Conclusion : Le traitement par l'Hexapeptide-2 permet de limiter les effets de la sénescence comme la diminution de l'expression du collagène I.

### Exemple 8 : Etude de l'activité de la β-galactosidase dans des fibroblastes humains rendus sénescents par sénescence réplicative, en présence d'Hexapeptide-2 (traitement long terme)

Le but de cette étude est de déterminer l'influence de l'Hexapeptide-2 sur des fibroblastes humains sénescents. Contrairement au modèle de sénescence obtenu par transfection de siRNA FOXO3a dont l'induction de la sénescence s'effectue en 48 heures, la sénescence réplicative est un processus long qui nécessite environ 3 mois. Les fibroblastes sont régulièrement subcultivés par trypsinisation afin de provoquer leur division et par conséquent de les faire vieillir.

Protocole : Des fibroblastes humains sont mis en culture dans un milieu spécifique et maintenus en culture (pendant plus de 27 passages), et traités ou non-par une application quotidienne d'Hexapeptide-2 à une concentration de 1 µM dans le milieu de culture (soit 5 applications par semaine). A chaque passage, une partie des cellules est congelée. Puis, des passages clés sont choisis : passages 4, 12, 16, 20, 24 et 27. Après décongélation, l'évaluation du phénotype sénescent des fibroblastes traités ou non avec l'Hexapeptide-2 à 1 µM est évaluée par le dosage de l'activité de la β-galactosidase.
Pour se faire, les cellules sont rincées et fixées dans un tampon de fixation (0.2% glutaraldéhyde, 2% formaldéhyde). Les cellules sont ensuite incubées, à 37°C sans CO₂ pendant 24 heures, avec une solution de X-Gal à 1 mg/ml dans 40 mM d'acide citrique/phosphate (pH 6), 5 mM K₃FeCN₆, 5 mM K₄FeCN₆, 150 mM NaCl and 2 mM MgCl₂. Les cellules sont alors examinées au microscope à lumière blanche (Nikon Eclipse E600 microscope).

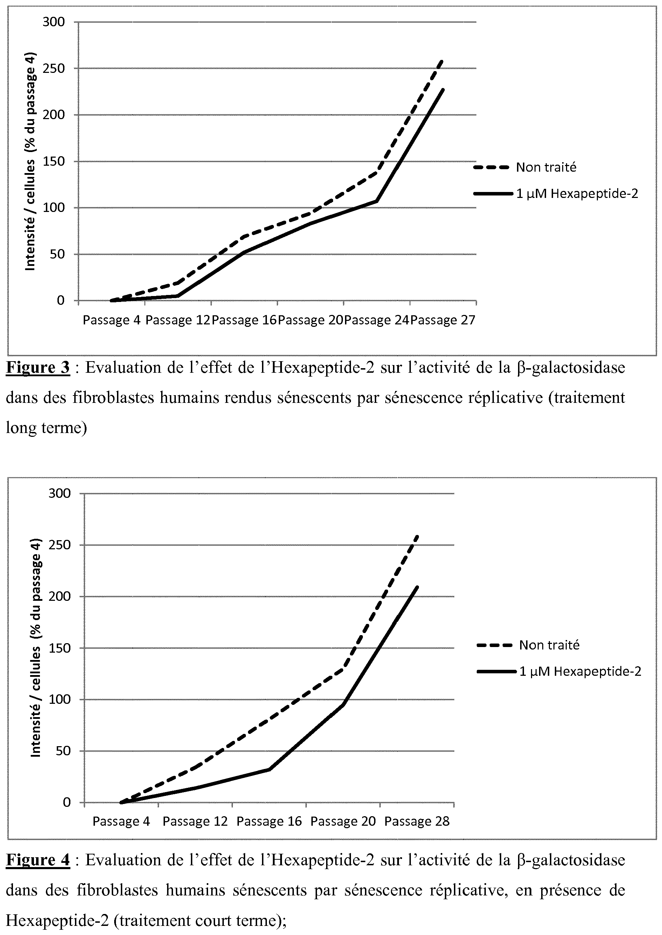

Résultats : Au fur et à mesure des passages cellulaires, l'activité de la β-galactosidase augmente, indiquant que le niveau de sénescence augmente à chaque passage. Lorsque ces cellules sont traitées avec l'Hexapeptide-2 à 1 µM, nous observons une diminution générale, significative, de -22% de l'activité de la beta-galactosidase en comparant aux cellules non traitées (selon le test *t* de Student). Les résultats sont montrés sur la figure 3.

Conclusion : Suite à un traitement à long terme, d'environ 3 mois, nous observons que les cellules traitées avec l'Hexapeptide-2 présentent un niveau de sénescence moindre que les cellules non traitées. Ainsi, le traitement avec l'Hexapeptide-2 permettrait de ralentir le vieillissement cellulaire.

### Exemple 9 : Etude de l'activité de la β-galactosidase dans des fibroblastes humains sénescents par sénescence réplicative, en présence d'Hexapeptide-2 (traitement court terme)

Le but de cette étude est de déterminer l'influence d'Hexapeptide-2 sur des fibroblastes humains sénescents par sénescence réplicative.

Protocole : Des fibroblastes humains sont mis en culture dans un milieu spécifique et maintenus en culture en traitement long (pendant plus de 28 passages, soit environ 3 mois). A chaque passage, une partie des cellules est congelée. Puis, des passages clés sont choisis : passages 4, 12, 16, 20 et 28. Après décongélation, les fibroblastes sont traités avec l'Hexapeptide-2 à 1 µM dans du milieu de culture, pendant 48 heures (2 applications par jour). L'évaluation du phénotype sénescent des fibroblastes traités ou non avec l'Hexapeptide-2 à 1 µM est évaluée par le dosage de l'activité de la β-galactosidase.
Pour se faire, les cellules sont rincées et fixées dans un tampon de fixation (0.2% glutaraldéhyde, 2% formaldéhyde). Les cellules sont ensuite incubées, à 37°C sans CO₂ pendant 24 heures, avec une solution de X-Gal à 1 mg/ml dans 40 mM d'acide citrique/phosphate (pH 6), 5 mM K₃FeCN₆, 5 mM K₄FeCN₆, 150 mM NaCl and 2 mM MgCl₂. Les cellules sont alors examinées au microscope à lumière blanche (Nikon Eclipse E600 microscope).

Résultats : Au fur et à mesure des passages cellulaires, l'activité de la β-galactosidase augmente, indiquant le niveau de sénescence de chaque passage. Lorsque ces cellules, rendues sénescentes sont traitées avec l'Hexapeptide-2 à 1 µM, nous observons une diminution générale, significative, de -31% de l'activité de la β-galactosidase en comparant aux cellules non traitées (selon le test t de Student). Les résultats sont présentés sur la figure 4

Conclusion : Suite à un traitement court terme, de 48 heures, nous observons que les cellules traitées avec Hexapeptide-2 présentent un niveau de sénescence moindre que les cellules non traitées. Ainsi, le traitement avec Hexapeptide-2 permettrait de ralentir le vieillissement cellulaire.

### Exemple 10 : Etude de l'activité de la prolidase dans des fibroblastes humains rendus sénescents par sénescence réplicative, en présence d'Hexapeptide-2 (traitement long terme)

Le but de cette étude est de déterminer l'influence de l'Hexapeptide-2 sur l'activité de la prolidase dans des fibroblastes humains sénescents. Il a été démontré, dans la littérature, que l'activité de la prolidase diminue avec le vieillissement cellulaire, et ceci est corrélé avec la diminution de collagène I observée dans les cellules sénescentes (Palka et al. Tokai J Exp Clin Med 1996)

Protocole : Des fibroblastes humains sont mis en culture dans un milieu spécifique et maintenus en culture en traitement long (pendant plus de 20 passages, soit environ 3 mois), traités ou non à l'aide d'une application quotidienne d'Hexapeptide-2 à une concentration de 1 µM dans du milieu de culture, (5 applications par semaine). A chaque passage, une partie des cellules est congelée. Puis, deux passages sont choisis : passages 12 et 20. Après décongélation, l'activité de la prolidase est observée pour ces conditions.
Pour se faire, les cellules sont détachées de leur support avec une solution de NaCl à 150 mM. Après avoir récupéré le culot cellulaire et activé la prolidase par une solution contenant du 2 mM de MnCl₂ pendant 2 heures à 37°C, le substrat de la prolidase est ajouté : 47 mM de Glycine-Proline (Sigma, Réf. G3002) pendant 1 heure à 37°C. Puis, le taux de proline contenu dans chaque échantillon est déterminé à une longueur d'onde de 515 nm par le réactif de Chinard. En parallèle, une gamme de concentration de proline est effectuée permettant l'établissement d'une courbe de calibration. Le taux de protéine est déterminé en utilisant le kit dosage de protéine BCA (Thermo Scientific, Réf. 23225), permettant de reporter le taux de proline sur le nombre de protéines de chaque échantillon.

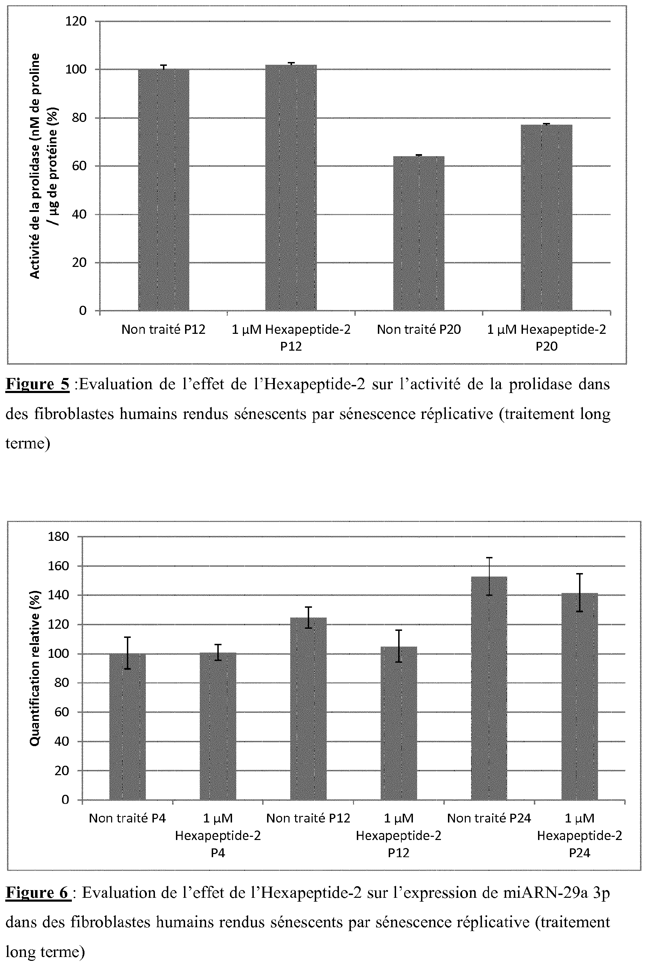

Résultat : la comparaison des passages P12 (cellules considérées comme jeunes) et P20 (cellules considérées comme sénescentes) montrent que l'activité de la prolidase diminue de 36% (valeur significative selon le test *t* de Student) lors de la sénescence réplicative. De manière intéressante, alors que l'Hexapeptide-2 a peu ou pas d'effet sur les cellules jeunes (P12), l'application d'Hexapeptide-2 sur les cellules sénescentes (P20) montre une restauration de 20% (valeur significative selon le test *t* de Student).de l'activité de la prolidase par rapport aux cellules témoins non traitées, au même passage.
Les résultats sont montrés sur la figure 5.

Conclusion : Lors de la sénescence, l'activité de la prolidase diminue, ainsi que l'expression du collagène. Le traitement avec l'Hexapeptide-2 permettrait de maintenir l'activité de la prolidase, entrainant ainsi un maintien du recyclage du collagène.

### Exemple 11 : Etude de l'expression de miARN-29a 3p dans des fibroblastes humains rendus sénescents par sénescence réplicative, en présence d'Hexapeptide-2 (traitement long terme)

Le but de cette étude est de déterminer l'influence de l'Hexapeptide-2 sur l'expression de miARN-29a 3p dans des fibroblastes humains sénescents, par sénescence réplicative. Les miARNs jouent un rôle fondamental dans la régulation post-transcriptionnelle de leurs cibles en les inhibant. Il a été démontré, dans la littérature, que l'expression du miARN-29a augmente avec la sénescence (Mancini M. *et al*., 2014). Grâce à des bases de données telles que miRanda, les cibles du miARN-29a ont été identifiées dont le collagène I et la prolidase.

Protocole : Des fibroblastes humains sont mis en culture dans un milieu spécifique et maintenus en culture en traitement à long terme (pendant plus de 24 passages, soit environ 3 mois), traités ou non à l'aide d'une application quotidienne d'Hexapeptide-2 à une concentration de 1 µM dans du milieu de culture, (5 applications par semaine). A chaque passage, une partie des cellules est congelée. Puis, trois passages sont choisis : passages 4, 12 et 24. Après décongélation, l'expression de miARN-29a 3p est observée pour ces conditions, par PCR quantitative.
Les miARNs sont extraits à l'aide d'un kit d'extraction (Ambion, Réf. AM1561), puis reverse transcrit avec un kit spécifique (Applied Biosystem, Réf. 4374966). Une PCR en temps réel est réalisée dans un thermocycleur à l'aide d'un *TaqMan Gene Expression Assays* spécifique du miARN-29a 3p (Applied Biosystem, 002112) et d'un *TaqMan Gene Expression Assays* spécifique du RNU44 utilisé comme contrôle endogène (Applied Biosystem, 001094). La quantification relative de l'expression du miARN-29a 3p est réalisée par la méthode comparative des Ct.

Résultat : Suite à la comparaison de trois passages P4, P12 et P20, nous pouvons observer que l'expression de miARN-29a 3p augmente significativement de +25% (entre P4 et P12) et +52% (entre P4 et P24) (test *t* de Student) avec la sénescence. A passage 4, aucun effet de l'Hexapeptide-2 n'a été observé sur l'expression de miARN-29a 3p. Par contre, aux passages 12 et 24, les cellules traitées avec l'Hexapeptide-2 montrent, respectivement, une diminution significative de -16% et de -7% de l'expression de miARN-29a 3p (selon le test *t* de Student). Les résultats sont montrés sur la figure 6.

Conclusion : Les cellules traitées avec l'Hexapeptide-2 à 1 µM semblent maintenir un certain niveau d'expression de miARN-29a 3p malgré la sénescence, permettant donc de limiter l'impact négatif de son expression sur les cibles telles que le collagène et la prolidase.

### Exemple 12 : Préparation d'une composition cosmétique anti-âge pour le visage

| **Noms commerciaux** | **Noms INCI** | **% massique** |
|---|---|---|
| **PHASE A** | | |
| **Eau Déminéralisée** | Aqua | 76,80 |
| **Na4 EDTA** | Tetrasodium EDTA | 0,05 |
| **Lubrasil™ II DM** | Glycerin & Glyceryl Acrylate/ Acrylic Acid Copolymer & Laureth-23 & Dimethicone | 3,00 |
| **Liquapar MEP** | Phenoxyethanol & Methylparaben & Ethylparaben & Propylparaben | 1,00 |

| **PHASE B** | | |
|---|---|---|
| **UltraThix® P-100** | Acrylic Acid/VP Crosspolymer | 0,60 |

| **PHASE C** | | |
|---|---|---|
| **NaOH Pearls** | Sodium Hydroxyde | 0,02 |
| **Eau Déminéralisée** | Aqua | 0,50 |

| **PHASE D** | | |
|---|---|---|
| **Belsil SDM 6022** | Stearoxy Dimethicone & Dimethicone | 2,00 |
| **Simulsol 165** | PEG-100 Stearate & Glyceryl Stearate | 2,00 |
| **Refined Shea Butter** | Butyrospermum Parkii (Shea) Butter | 2,00 |
| **Ceraphyl® 28** | Cetyl Lactate | 1,50 |
| **Ceraphyl® 791** | Isocetyl Stearoyl Stearate | 2,00 |
| **Ceraphyl® ODS** | Octyldodecyl Stearate | 3,00 |
| **Ceraphyl® 368** | Ethylhexyl Palmitate | 4,00 |

| **PHASE E** | | |
|---|---|---|
| **NaOH Pearls** | Sodium Hydroxyde | 0,03 |
| **Eau Déminéralisée** | Aqua | 0,50 |

| **PHASE F** | | |
|---|---|---|
| **Hexapeptide-2** | | 1,00 |
| | | ***100,00*** |

Dans les conditions de préparation de cette formule, l'Hexapeptide-2 à 1 % correspond à une concentration de 1 µM.

La composition cosmétique pour le visage dite « placebo » est réalisée exactement de la même manière avec l'exception que l'Hexapeptide-2 est remplacé par de l'eau purifiée.

### Exemple 13 Etude de l'effet de l'Hexapeptide-2 à 1 % sur l'apparence des rides ( étude clinique)

Objectif : étude clinique sur l'apparence des rides après traitement topique par une composition cosmétique comprenant de l'Hexapeptide-2 à 1 %, contre placebo.

### Protocole :

Nombre de volontaires : 35 âgés (âgés de 35 à 65 ans).
Etude : étude en double aveugle contre placebo.
Produits testés : Hexapeptide-2 formulé à 1% (soit une concentration finale de 1 µM) dans une composition cosmétique décrite dans l'exemple 12 *versus* la composition placebo. Dans les conditions du
Nombre d'application : 2 applications par jour, matin et soir, à une dose de 2 mg/cm².
Durée du test : 6 semaines.
Visites de contrôle : Jour 0 (J0), et jour 42 (J42).
Mesures : analyse des répliques de silicone faites au niveau de la patte d'oie à l'aide du logiciel Visioline® VL650
Analyses statistiques : en fonction de la normalité de distribution des données, le test " t " de Student ou le test de Wilcoxon ont été utilisés.

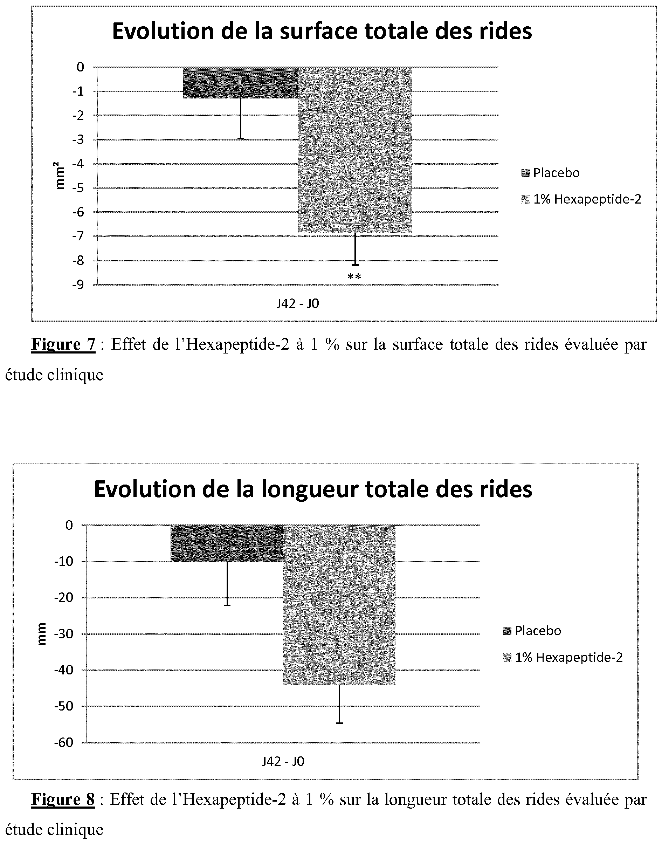

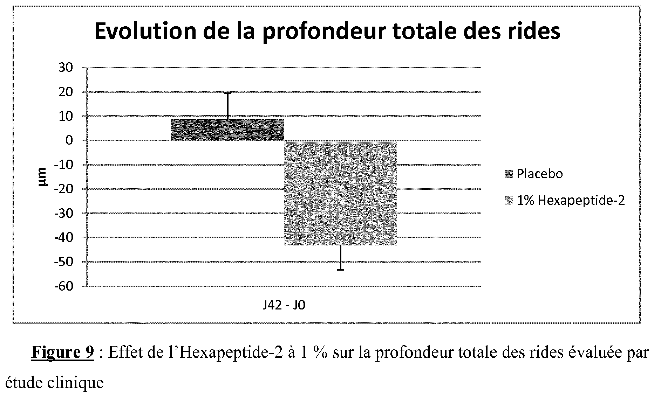

Résultats : Après application d'Hexapeptide-2 formulé à 1% dans la composition selon l'exemple 12, l'analyse des répliques de silicone montrent une diminution statistiquement significative à J42 du nombre, de la surface totale, de la longueur totale, de la profondeur totale et maximale des rides, comparé au placebo. Les résultats sont présentés sur les figures 7, 8 et 9 et dans le tableau 5 ci-dessous.

| Tableau 5 | Traitement | Temps | Moyenne | sem | p | % d'évolution | % de volontaires améliorés |
|---|---|---|---|---|---|---|---|
| Nombre de rides | Placebo | J42 - J0 | -7.83 | 14.97 | 0.0206* | -12.47% | 57% |
| | 1% Hexapeptide-2 | J42 - J0 | -53.14 | 17.36 | | | |
| Surface totale des rides (mm²) | Placebo | J42 - J0 | -1.29 | 1.64 | 0.0093** | -27.59% | 63% |
| | 1% Hexapeptide-2 | J42 - J0 | -6.85 | 1.33 | | | |
| Longueur totale (mm) | Placebo | J42 - J0 | -10.17 | 11.84 | 0.0281* | -17.48% | 66% |
| | 1% Hexapeptide-2 | J42 - J0 | -43.99 | 10.62 | | | |
| Profondeur totale (µm) | Placebo | J42 - J0 | -364.44 | 716.25 | 0.0088** | -15.75% | 60% |
| | 1% Hexapeptide-2 | J42 - J0 | -3030.34 | 850.01 | | | |
| Profondeur maximale (µm) | Placebo | J42 - J0 | 8.68 | 10.81 | 0.0021** | -23.13% | 63% |
| | 1% Hexapeptide-2 | J42 - J0 | -43.06 | 10.29 | | | |

| | | | | | | | |
|---|---|---|---|---|---|---|---|
| * : significatif; ** : très significatif, avec le test de Student ou le test de Wilcoxon (test sélectionné en fonction de la normalité de distribution des données); moyenne n=35 +/- sem. | | | | | | | |

Conclusions : L'application topique d'Hexapeptide-2 à 1 % (soit à la concentration de 1 µM) pendant 42 jours provoque une diminution statistiquement significative de l'apparence des rides.

## Revendications

1. Utilisation cosmétique d'une composition comprenant, en tant qu'agent actif, entre 0,1 µM et 1 µM d'un peptide de synthèse de séquence (SEQ ID N°1) His-D-Trp-Ala-Trp-D-Phe-Lys-NH2 ou un de ses sels, dans un milieu physiologiquement adapté, pour diminuer ou retarder l'apparition de la sénescence cellulaire et des signes du vieillissement cutané choisis parmi l'amincissement de la peau, le relâchement, la perte d'hydratation, l'atonie, les rides profondes et les ridules, la perte de fermeté et de tonicité, l'atrophie dermique à l'exception des anomalies pigmentaires de la peau, la composition étant appliquée par voie topique, au moins une fois par jour, pendant une durée d'au moins 2 jours.

2. Utilisation cosmétique selon la revendication 1, dans laquelle la composition comprend entre 0,5 et 1 µM dudit peptide de synthèse.

3. Utilisation cosmétique selon la revendication 1, dans laquelle la composition comprend moins de 1 µM dudit peptide de synthèse.

4. Utilisation cosmétique selon l'une des revendications 1 à 3, dans laquelle l'application a lieu 2 fois par jour.

5. Utilisation cosmétique selon l'une des revendications 1 à 3, dans laquelle, l'application a lieu pendant une durée d'au moins 6 semaines.

6. Utilisation cosmétique selon l'une des revendications 1 à 3, dans laquelle, l'application a lieu pendant une durée d'au moins 3 mois.

7. Utilisation cosmétique selon l'une des revendications 1 à 6, dans laquelle ledit peptide de synthèse augmente l'activité de la prolidase et la synthèse du collagène.

8. Utilisation cosmétique selon la revendication 1 à 7, dans laquelle les signes du vieillissement cutané sont les rides.

9. Utilisation cosmétique selon la revendication 1 à 8, dans laquelle la composition comprend en outre au moins un autre agent actif destiné à renforcer l'action de l'agent actif selon l'invention, choisi parmi les peptides, la vitamine C et ses dérivés, les vitamines du groupe B, la DHEA, les phytostérols, l'acide salicylique et ses dérivés, les rétinoïdes, les flavonoïdes, les amines de sucres, les azoles, les sels métalliques, les extraits peptidiques d'origine végétale ou encore les polymères.

## Patentansprüche

1. Kosmetische Verwendung einer Zusammensetzung, umfassend, als aktiven Bestandteil, zwischen 0,1 µM und 1 µM eines synthetischen Peptids mit der Sequenz (SEQ ID Nr. 1) His-D-Trp-Ala-Trp-D-Phe-Lys-NH2 oder eines seiner Salze, in einem physiologisch geeigneten Medium, um das Auftreten der Zellalterung und von Anzeichen der Hautalterung zu verringern oder zu verzögern, ausgewählt aus Ausdünnung der Haut, Erschlaffung, Feuchtigkeitsverlust, Atonie, tiefen Falten und Fältchen, Festigkeits- und Tonizitätsverlust, Hautatrophie mit Ausnahme von Pigmentanomalien der Haut, wobei die Zusammensetzung auf topischem Weg, mindestens einmal täglich, während einer Dauer von mindestens 2 Tagen aufgebracht wird.

2. Kosmetische Verwendung nach Anspruch 1, wobei die Zusammensetzung zwischen 0,5 und 1 µM des synthetischen Peptids umfasst.

3. Kosmetische Verwendung nach Anspruch 1, wobei die Zusammensetzung weniger als 1 µM des synthetischen Peptids umfasst.

4. Kosmetische Verwendung nach einem der Ansprüche 1 bis 3, wobei das Aufbringen 2-mal täglich erfolgt.

5. Kosmetische Verwendung nach einem der Ansprüche 1 bis 3, wobei das Aufbringen während einer Dauer von mindestens 6 Wochen erfolgt.

6. Kosmetische Verwendung nach einem der Ansprüche 1 bis 3, wobei das Aufbringen während einer Dauer von mindestens 3 Monaten erfolgt.

7. Kosmetische Verwendung nach einem der Ansprüche 1 bis 6, wobei das synthetische Peptid die Aktivität von Prolidase und die Synthese von Kollagen erhöht.

8. Kosmetische Verwendung nach einem der Ansprüche 1 bis 7, wobei die Anzeigen der Hautalterung Falten sind.

9. Kosmetische Verwendung nach einem der Ansprüche 1 bis 8, wobei die Zusammensetzung außerdem mindestens ein anderes aktives Mittel umfasst, das dazu bestimmt ist, die Wirkung des aktiven Mittels gemäß der Erfindung zu verstärken, ausgewählt aus Peptiden, Vitamin C und seinen Derivaten, Vitaminen der Gruppe B, DHEA, Phytosterinen, Salicylsäure und ihren Derivaten, Retinoiden, Flavonoiden, Aminozuckern, Azolen, Metallsalzen, Peptidextrakten pflanzlichen Ursprungs oder auch Polymeren.

## Claims

1. The cosmetic use of a composition comprising, as the active agent, between 0.1 µM and 1 µM of a synthesized peptide with the sequence His-D-Trp-Ala-Trp-D-Phe-Lys-NH2 (SEQ ID N°1) or one of its salts, in a suitable physiological medium, to reduce or delay the appearance of cell senescence and signs of skin aging selected from thinning of the skin, sagging, loss of moisture, atony, deep wrinkles and fine lines, loss of firmness and of tone, dermal atrophy, with the exception of pigmentary anomalies of the skin, the composition being applied topically, at least once per day, for a period of at least two days.

2. The cosmetic use as claimed in claim 1, in which the composition comprises between 0.5 and 1 µM of said synthesized peptide.

3. The cosmetic use as claimed in claim 1, in which the composition comprises less than 1 µM of said synthesized peptide.

4. The cosmetic use as claimed in one of claims 1 to 3, in which the application is carried out twice a day.

5. The cosmetic use as claimed in one of claims 1 to 3, in which the application is carried out for a period of at least six weeks.

6. The cosmetic use as claimed in one of claims 1 to 3, in which the application is carried out for a period of at least three months.

7. The cosmetic use as claimed in one of claims 1 to 6, in which said synthesized peptide increases the activity of prolidase and of the synthesis of collagen.

8. The cosmetic use as claimed in one of claims 1 to 7, in which the signs of skin aging are wrinkles.

9. The cosmetic use as claimed in one of claims 1 to 8, in which the composition further comprises at least one other active agent intended to reinforce the action of the active agent in accordance with the invention, selected from peptides, vitamin C and its derivatives, the vitamins from group B, DHEA, phytosterols, salicylic acid and its derivatives, retinoids, flavonoids, sugar amines, azoles, metal salts, peptide extracts of plant origin, and from polymers.


### SEQUENCE LISTING

<110> ISP Investments INC.
<120> NOUVELLES UTILISATIONS DU PEPTIDE DE SEQUENCE HIS-D-TRP-ALA-TRP-D-PHE-LYS-NH2 POUR DIMINUER OU RETARDER L'APPARITION DE LA SENESCENCE CELLULAIRE ET DES SIGNES DU VIEILLISSEMENT CUTANE
<130> Bv 15-176 PCT
<150> FR 1553139
   <151> 2015-04-10
<160> 1
<170> PatentIn version 3.5
<210> 1
   <211> 6
   <212> PRT
   <213> Artificial Sequence
<220>
   <223> synthetic peptide
<220>
   <221> MISC_FEATURE
   <222> (2)..(2)
   <223> Configuration D
<220>
   <221> MISC_FEATURE
   <222> (5)..(5)
   <223> Configuration D
<220>
   <221> MOD_RES
   <222> (6)..(6)
   <223> AMIDATION
<400> 1
   His Trp Ala Trp Phe Lys
   1               5